# EUROPÄISCHE PATENTSCHRIFT

(11) **EP 1 183 003 B1**
(45) Veröffentlichungstag und Bekanntmachung des Hinweises auf die Patenterteilung: **23.02.2005**
(21) Anmeldenummer: 00936700.4
(22) Anmeldetag: 28.04.2000
(51) Int. Cl.: A61K 7/00, A61K 7/32, A61K 7/34, A61K 7/36, A61K 7/38

(54) **MIT TENSIDEN UMHÜLLTE ODER IMPRÄGNIERTE ANTITRANSPIRANT-WIRKSTOFFE**
ANTIPERSPIRANT ACTIVE INGREDIENTS IMPREGNATED OR COATED WITH SURFACTANTS
AGENTS ACTIFS ANTITRANSPIRANTS IMPREGNES OU ENROBES DE TENSIOACTIFS

(30) Priorität: 07.05.1999 DE 19921184
(43) Veröffentlichungstag der Anmeldung: 06.03.2002
(73) Patentinhaber: Henkel Kommanditgesellschaft auf Aktien, 40589 Düsseldorf-Holthausen (DE)
(72) Erfinder: BANOWSKI, Bernhard, D-40597 Düsseldorf (DE); WADLE, Armin, D-40699 Erkrath (DE); HENGSTERMANN, Dieter, D-46325 Borken (DE)
(86) Internationale Anmeldenummer: PCT/EP2000/003864
(87) Internationale Veröffentlichungsnummer: WO 2000/067703

(56) Entgegenhaltungen:
- EP-A- 0 388 111
- EP-A- 0 936 267
- WO-A-94/13255
- DE-A- 19 816 475
- GB-A- 1 109 840
- US-A- 4 352 789
- US-A- 4 524 062
- US-A- 5 320 828
- US-A- 5 486 355
- DATABASE WPI Section Ch, Week 197530 Derwent Publications Ltd., London, GB; Class D25, AN 1975-50297W XP002154662 & JP 50 018482 B (KOBAYASHI T), 30. Juni 1975 (1975-06-30)

## Beschreibung

Die Erfindung betrifft schweißhemmende Zubereitungen feinteiliger adstringierender Aluminium-, Zirkonium- oder Zinksalze, die mit einem wasserlöslichen Tensid umhüllt oder imprägniert sind.

Schweißhemmende Zubereitungen enthalten üblicherweise ein adstringierendes Material in einem geeigneten Träger. Als Träger können wäßrige oder wäßrig-alkoholische Lotionen, wasserfreie Aerosole, Roll-On-Suspensionen, Cremes oder Stifte verwendet werden.

Wasserfreie Zubereitungen enthalten die adstringierenden Metallsalze, üblicherweise Aluminiumsalze, als Dispersion in einem flüssigen Träger, der im Falle der Stiftzubereitungen durch Gelier- oder Festigungsmittel zu einem Stift verfestigt ist.

Als flüssige Träger können allgemein flüssige Ölkomponenten, z.B. Kohlenwasserstoffe, Silikonöle, Esteröle und andere flüssige Öle, z.B. verzweigte Fettalkohole, fungieren, bevorzugt solche mit Siedepunkten unterhalb 200° C oder mit einer gewissen Flüchtigkeit, z.B. niedermolekulare lineare und cyclische Siloxane (Cyclomethicone) und deren Gemische mit anderen polaren und unpolaren Ölkomponenten.

Um die Antitranspirantleistung zu verbessern, hat man die adstringierenden Aluminiumsalze z.B. gemäß EP 295070 A1 oder EP 404533 A1 in mehrwertigen Alkoholen und Wasser solubilisiert eingesetzt. Diese Verfahren erschweren aber die Formulierung von wasserfreien Antitranspirant-Stiften und führen zu klebrigen oder schmierigen Rückständen auf der Haut. Aus WO 9413255 waren adstringierende Aluminium- und Zirkoniumsalze bekannt, die mit einem hydrophilen Polymerisat, z.B. einem Polyethylenglycol, beschichtet sind, um störende Reaktionen mit basischen Komponenten zu verhindern.

US-A-5 320 828 offenbart wasserfreie Antitranspirantzusammensetzungen, wobei als Wirkstoff feinteilige adstringierende Aluminiumsalze, die mit einer Beschichtung aus Carbonsäuren oder Carbonsäurederivaten versehen sind, eingesetzt werden.

Man hat auch den wasserfreien Antitranspirant-Stiften nichtionische Tenside zugesetzt, dies aber mit dem Ziel, die Abwaschbarkeit von Rückständen von der Haut und von Kleidungsstücken zu erleichtern.

Es bestand daher die Aufgabe, schweißhemmende Zubereitungen zu entwickeln, die als wasserfreie Dispersionen der adstringierenden Wirkstoffe, sei es als Lotion, Aerosol, Roll-On, Creme oder Stift, formuliert sind und eine rasche und effektive Antitranspirant-Wirkung aufweisen.

Die Lösung dieser Aufgabe gelang durch eine Umhüllung oder Imprägnierung der teilchenförmigen adstringierenden Aluminium-, Zirkonium- oder Zinksalze mit einem wasserlöslichen nichtionischen, zwitterionischen oder kationischen Tensid.

Gegenstand der vorliegenden Patentanmeldung ist eine schweißhemmende Zubereitung feinteiliger adstringierender Aluminium-, Zirkonium- oder Zinksalze, deren Teilchen eine mittlere Teilchengröße von weniger als 100 µm aufweisen und mit 0,1 - 10 Gew.-%, bezogen auf die adstringierenden Aluminium-, Zirkonium- und Zinksalze, eines wasserlöslichen nichtionischen, zwitterionischen oder kationischen Tensids imprägniert oder umhüllt sind.

Adstringierende Aluminium-, Zirkonium- oder Zinksalze, die sich als antitranspirative Wirkstoffe eignen, sind z.B. Aluminiumchlorid, Aluminiumhydroxychloride, Aluminiumdichlorhydrat, Aluminiumsesquichlorhydrat, deren Komplexverbindungen mit z.B. 1,2-Propylenglycol, Aluminiumhydroxyallantoinat, Aluminiumchlorid-tartrat, Aluminium-Zirkonium-Trichlorhydrat, Aluminium-Zirkonium-tetrachlorhydrat, Aluminium-Zirkoniumpentachlorhydrat und deren Komplexverbindungen mit Aminosäuren, z.B. mit Glycin. Eine besonders bevorzugte Verbindung für diesen Zweck ist z.B. Aluminiumchlorhydrat der Zusammensetzung Al₂(OH)₅Cl•n H₂O, worin n = 2 oder 3 ist.

Weitere geeignete adstringierende Salze sind z.B. Aluminium-Zirkonium-tetrachlor-Glycin-Komplex, Natrium-aluminium-lactat, Kalium-aluminium-sulfat (K-Alaun), Natrium-Zirkonium-lactat, Aluminiumsulfat, Zinksulfat und Zn-Phenolsulfonat. Die adstringierenden Salze sollen feinteilig sein, ihre mittlere Teilchengröße sollte unter 100 µm, bevorzugt unter 50 µm liegen.

Als wasserlösliche Tenside eignen sich solche Tenside, die eine bevorzugt lineare lipophile Alkyl- oder Acylgruppe mit 8 - 18 C-Atomen und eine wasserlöslich-machende ionische Gruppe oder eine nichtionische Polyol- oder Polyethergruppe, bevorzugt am Ende der lipophilen Gruppe, aufweisen. Anstelle der lipophilen Alkyl- oder Acylgruppe kann auch eine lineare Dimethylpolysiloxan-Gruppierung enthalten sein. Als wasserlöslich werden dabei alle Tenside verstanden, die bei 20°C zu wenigstens 1 Gew.-% in Wasser löslich sind. Auch trüblösliche Tensidgemische oder Homologengemische gelten als wasserlöslich, wenn wenigstens 1 Gew.-% davon bei 20 °C in Wasser gelöst ist.

Geeignete nichtionische Tenside sind z.B. die Anlagerungsprodukte von 8 - 40 Mol Ethylenoxid und/oder 1 - 40 Mol Propylenoxid an lineare Fettalkohole mit 8 - 22 C-Atomen, an Fettsäuren mit 12 - 22 C-Atomen, an Fettsäuremonoglyceride, an Fettsäurealkanolamide, an Sorbitanfettsäureester, an Methylglucoside oder andere oxethylierbare Lipide. Auch Block-Copolymerisate aus Ethylenoxid und Propylenoxid (sog. EO/PO-Block-copolymere) sind geeignete nichtionische Tenside. Besonders bevorzugt für die Zwecke der vorliegenden Erfindung sind aber die sogenannten Alkylglycoside oder Alkyl(oligo)-glycoside geeignet, die z.B. durch Umsetzung von Glucose mit einwertigen Alkoholen unter Glucosidbindung oder durch Umacetalisierung aus Butyl-glucosid und längerkettigen Alkoholen zugänglich und im Handel erhältlich sind.

So ist als nichtionisches Tensid bevorzugt ein Alkyl-(oligo)-glycosid der Formel R-O-(G)ₙ, geeignet, in der R eine Alkylgruppe mit 8 - 22 C-Atomen, bevorzugt eine lineare Alkylgruppe mit 8 - 22 C-Atomen, und (G)ₙ der Rest eines Glucosids oder Oligoglucosids mit einem mittleren Oligomerisationsgrad, n von 1 - 10, bevorzugt von 1 - 2, ist. Solche Tenside sind im Handel z.B. unter der Bezeichnung Plantacare® 2000 (R = C₈ - C₁₆, n = 1,4) erhältlich.

Bevorzugt geeignete nichtionische Tenside sich auch die sogenannten Dimethicone - Copolyol-und Dimethicone Polyglucoside-Tenside, die an einer linearen Dimethylpolysiloxankette mit z.B. 4-6 Si(CH₃)₂O-Gruppen eine oder zwei Polyoxyalkylengruppen oder Glucosidreste gebunden enthalten. Solche Silikon-Tenside sind z.B. aus Cosmetics & Toileteries 98 (5/1983), 103-106 und aus Parfümerie und Kosmetik, 67 (3/1983), 148-154 bekannt.

Geeignete Handelsprodukte sind z.B. Abil EM97 (Goldschmidt), Dow coming 2501, Belsil 6031, 6032 und 6035 (Wacker), Wacker SPG 121 sowie Dow Corning 5200.

Weiterhin eignen sich auch zwitterionische Tenside, z.B. die Betaintenside, ampholytische Tenside und die polaren nichtionischen Tenside vom Typ der Alkylaminoxide.

Auch kationische wasserlösliche Tenside mit quartären Ammoniumgruppen können verwendet werden, wenn mit den übrigen Komponenten der schweißhemmenden Zubereitung keine Unverträglichkeit besteht.

Die Umhüllung (coating) oder Imprägnierung der adstringierenden Aluminium-, Zirkoniumoder Zink-Salze kann auf einfache Weise dadurch geschehen, daß man eine wäßrige Lösung mit einem Gehalt an 100 Gewichtsteilen des adstringierenden Salzes und 0,1 bis 10 Gewichtsteilen eines nichtionischen, wasserlöslichen Tensids entwässert und den Rückstand auf eine Teilchengröße von weniger als 100 µm zerkleinert. Die Entwässerung kann dabei durch Eindampfen, bevorzugt unter schonenden Bedingungen, z.B. unter vermindertem Druck, in der Wirbelschicht, durch Sprühtrocknung oder Lyophilisation erfolgen. Das Zerkleinern des Rückstandes kann, falls erforderlich, durch Mahlen erfolgen. Die Teilchengröße ist dabei als mittlere Teilchengröße (Gewichtsmittel) zu verstehen, bevorzugt sollten aber mehr als 90 Gew.-% des Teilchenmaterials einen Maximaldurchmesser von weniger als 100 µm aufweisen.

### Ein weiteres geeignetes Verfahren zur Herstellung der erfindungsgemäßen schweißhemmenden Zubereitung feinteiliper, adstringierender Aluminium-, Zirkonium- oder Zink-

Salze besteht darin, daß man ein bereits feinteiliges, adstringierendes Salz mit einer Teilchengröße von weniger als 100 µm mit einem flüssigen oder gelösten nichtionischen wasserlöslichen Tensid in einer Menge von 0,1 - 10 Gewichtsteilen des Tensids pro 100 Gewichtsteilen des Salzes besprüht und gegebenenfalls das Lösungsmittel verdampft.

Die meisten geeigneten nichtionischen Tenside sind bei Temperaturen unter 100°C flüssig und lassen sich im geschmolzenen Zustand feinteilig versprühen. Man kann aber auch z.B. konzentrierte wäßrige Lösungen mit z.B. 50 Gew.-% des gelösten Tensids auf die adstringierenden Salze aufdüsen und die damit eingetragene Feuchtigkeit durch Trocknung entfernen.

Die erfindungsgemäßen schweißhemmenden Zubereitungen eignen sich besonders gut als Aktivkomponente zur Herstellung von wasserfreien Antitranspirantien. In solchen Antitranspirantien sind die adstringierenden Salze in einem flüssigen, pastösen oder festen bzw. gelierten Träger dispergiert. Geeignete Träger können Aerosol-Sprays, Roll-On-Suspensionen, wasserfreie Cremes oder Stiftformulierungen sein.

Ein weiterer Gegenstand der Erfindung sind daher Antitranspirantien in Form wasserfreier Zusammensetzungen, worin 0,1 - 40 Gew.-%, bevorzugt 10 - 25 Gew.-%, einer schweißhemmenden Zubereitung feinteiliger adstringierender Aluminium-, Zirkonium- oder Zinksalze mit einer mittleren Teilchengröße von weniger als 100 µm, die mit 0,1 - 10 Gew.-%, bezogen auf die adstringierenden Aluminium-, Zirkonium- und Zinksalze, eines wasserlöslichen nichtionischen, zwitterionischen oder kationischen Tensids imprägniert oder umhüllt sind, dispergiert sind.

Die erfindungsgemäßen Antitranspirantien können darüber hinaus alle für solche Zubereitungen üblichen Hilfsmittel enthalten. Geeignete Hilfsmittel sind z.B. wasserfreie, unpolare Trägerflüssigkeiten wie z.B. Kohlenwasserstoffe oder Silikonöle, insbesondere solche mit niedrigen Siedepunkten oder mit erhöhter Flüchtigkeit. Besonders bevorzugt sind hierfür die cyclischen Siloxane wie z.B. Octamethyltetracyclosiloxan oder Decamethylpentacyclosiloxan. Weitere Komponenten sind z.B. kosmetische Ölkomponenten wie z.B. Fettsäureester wie z.B. Isopropylmyristat, Decyloleat oder Glycerintricaprylat, verzweigte Alkohole wie z.B. 2-Hexyl-decanol, 2-Octyl-dodecanol, Polypropylenglycol-Ether wie z.B. PPG-3-Myristylether oder PPG-14-Butylether. Weitere Hilfsmittel zur Stabilisierung der Dispersion sind z.B. feinteilige anorganische Silikate wie z.B. Talkum, Aerosil oder Schichtsilikate.

Auch Antioxidantien und vor allem Riechstoffe gehören zu den typischen Komponenten wasserfreier Antitranspirantien. Zur Formulierung von Aerosolen werden schließlich geeignete Treibgase, z.B. Propan, Butan, Isobutan, Dimethylether, Fluorkohlenwasserstoffe oder Gemische davon oder Gemische mit Kohlendioxid oder Stickoxidul (N₂O) verwendet.

Zur Formulierung stiftförmiger Zubereitungen ist es erforderlich, den flüssigen Träger mit den enthaltenen Ölkomponenten durch geeignete Hilfsmittel zu verfestigen oder zu gelieren. Für diesen Zweck eignen sich bevorzugt gesättigte Fett- oder Wachskomponenten mit Schmelzpunkten von 40 - 90°C. Geeignete Festigungsmittel sind z.B. gesättigte, lineare Fettalkohole mit 14 - 40 C-Atomen, Wachsester wie z.B. Cetylpalmitat, Stearylstearat, gesättigte Triglyceride wie z.B. Tripalmitin, gehärtetes Palmfett oder gehärtetes Rizinusöl. Auch Calciumseifen von C₁₂ -C₂₂-Fettsäuren oder von Hydroxystearinsäure können zur Gelierung verwendet werden. Geeignete Geliermittel sind auch z.B. Dibenzylidensorbit und N-Acylaminosäureamide.

Eine besonders bevorzugte erfindungsgemäße Antitranspirant-Zusammensetzung in Form eines Stiftes enthält

| | |
|---|---|
| 10 - 60 Gew.-% | einer unpolaren Trägerflüssigkeit, |
| 5 - 30 Gew.-% | einer festigenden Fett- oder Wachskomponente sowie |
| 10 - 25 Gew.-% | einer erfindungsgemäßen schweißhemmenden Zubereitung feinteiliger adstringierender Aluminium-, Zirkonium- oder Zinksalze, deren Teilchen eine mittlere Teilchengröße unter 100 µm aufweisen und mit 0,1-10 Gew.-%, bezogen auf die adstringierenden Aluminium-, Zirkonium- und Zink- salze, eines nichtionischen Tensids, bevorzugt eines Alkyl-(oligo)-glucosids, imprägniert oder umhüllt sind. |

Zur Herstellung solcher erfindungsgemäßer stiftförmiger Antitranspirant-Zusammensetzungen wird die festigende Fett- oder Wachskomponente in der unpolaren Trägerflüssigkeit unter Erwärmen gelöst; bei ca. 60 - 90°C wird dann die erfindungsgemäß beschichtete schweißhemmende Salzzubereitung eingemischt. Sodann werden die übrigen Komponenten, z.B Ölkomponenten, Tenside, Talkum eingemischt und während der Abkühlphase der Duftstoff zugesetzt. Vor dem Erstarren wird die Masse in vorbereitete Stifthülsen gegossen.

Die folgenden Beispiele sollen den Erfindungsgegenstand näher erläutern:

### Beispiele

### 1. Herstellung einer erfindungsgemäßen Aluminium-hydroxychlorid(AHC)-Zubereitung

### 1.1. mit 5 Gew.-% Alkylglucosid

40,0 g Micro Dry® SUF Pulver (Interorgana/Reheis) und 4 g Plantacare® 2000 wurden vollständig in 100 g Wasser gelöst. Anschließend wurde die Lösung zur Trockene eingeengt. Der Rückstand wurde bei 95°C im Wärmeschrank 90 Min. getrocknet und nach Abkühlung auf 20°C in der Reibschale feingemahlen. Das Mahlgut wurde durch ein Sieb (Maschenweite 40 µm) von Grobanteilen befreit.

### 1.2.

98g Locron®L (Clariant) und 2 g Plantacare®2000 wurden bei Raumtemperatur gemischt und die erhaltene Lösung bei vermindertem Druck zur Trockene eingedampft. Der Rückstand wurde im Mörser vermahlen und durch ein Sieb mit 40 µm Maschenweite gesiebt.

### 1.3. mit 1 Gew.-% Alkylglucosid

49,5 g Micro Dry® Ultrafine (Interorgana/Reheis) wurden mit 1,0 g Plantacare® 2000 unter Rühren besprüht. Das feuchte Pulver wurde unter Rühren im Vakuum bei 40°C getrocknet.

### 2. Anwendungsbeispiele

### 2.1. Antitranspirantspray

| | |
|---|---|
| AHC-Zubereitung nach Beispiel 1.1 | 10,0 Gew.-% |
| Isopropylpalmitat | 5,0 Gew.-% |
| Dow Corning® 244 Fluid | 5,0 Gew.-% |
| Parfümöl | 0,5 Gew.-% |
| Treibgas-Gemisch | 79,5 Gew.-% |

### 2.2. Antitranspirant-Roll-On

| | |
|---|---|
| AHC-Zubereitung nach Beispiel 1.2 | 20,0 Gew.-% |
| Eutanol® G | 1,0 Gew.-% |
| Aerosil® 200 | 1,0 Gew.-% |
| Dow Corning® 245 Fluid | 77,0 Gew.-% |
| Parfümöl | 1,0 Gew.-% |

### 2.3. Antitranspirant-Sift

| | |
|---|---|
| AHC- Zubereitung nach Beispiel 1.1 | 20,0 Gew.-% |
| Dow Corning® 245 Fluid | 30,0 Gew.-% |
| Cetiol® OE | 10,0 Gew.-% |
| Ucon Fluid® AP | 5,0 Gew.-% |
| Cutina® HR | 5,0 Gew.-% |
| Stearylalkohol | 20,0 Gew.-% |
| Talkum | 10,0 Gew.-% |

Es wurden die folgenden Handelsprodukte verwendet:

| | |
|---|---|
| Dow Corning® 244 Fluid | Octamethyl-Cyclotetrasiloxan |
| Dow Corning® 245 Fluid | Decamethyl-Cyclopentasiloxan |
| Eutanol® G | 2-Octyl-dodecanol |
| Cetiol® OE | Di-n-octylether |
| Ucon Fluid® AP | PPG 14-monobutylether |
| Cutina® HR | gehärtetes Rizinusöl |
| Aerosil® 200 | disperses Siliciumoxid |

## Patentansprüche

1. Schweißhemmende Zubereitungen feinteiliger adstringierender Aluminium-, Zirkoniumoder Zink-Salze, **dadurch gekennzeichnet, daß** die Teilchen eine mittlere Teilchengröße von weniger als 100 µm ausweisen und mit 0,1 - 10 Gew.-%, bezogen auf die adstringierenden Aluminium-, Zirkonium- und Zinksalze, eines wasserlöslichen nichtionischen, zwitterionischen oder kationischen Tensids imprägniert oder umhüllt sind.

2. Schweißhemmende Zubereitungen gemäß Anspruch 1, **dadurch gekennzeichnet, daß** als Aluminiumsalz das Aluminium-hydroxychlorid der Formel Al₂(OH)₅Cl•nH₂O, in der n = 2 oder 3 ist, enthalten ist.

3. Schweißhemmende Zubereitungen gemäß Anspruch 1, **dadurch gekennzeichnet, daß** das Tensid ein Alkyl-(oligo)-glycosid der Formel R-O-(G)ₙ, ist, in der R eine Alkylgruppe mit 8 - 22 C-Atomen und (G)ₙ der Rest eines Glucosids oder Oligo-glucosids mit einem mittleren Oligomerisationsgrad n von 1-10 ist.

4. Schweißhemmende Zubereitungen gemäß Anspruch 1, **dadurch gekennzeichnet, daß** das Tensid ein Dimethicone-Copolyol-Tensid oder ein Dimethicone-Polyglucosid-Tensid ist, das an einer linearen Dimethylpolysiloxankette eine oder zwei Polyoxyalkylengruppen oder Glucosidreste gebunden enthält.

5. Verfahren zur Herstellung einer schweißhemmenden Zubereitung feinteiliger adstringierender Aluminium-, Zirkonium- oder Zink-Salze **dadurch gekennzeichnet, daß** man eine wäßrige Lösung mit einem Gehalt von 100 Gewichtsteilen eines adstringierenden Salzes und 0,1 bis 10 Gewichtsteilen eines nichtionischen Tensids entwässert und den Rückstand auf eine mittlere Teilchengröße von weniger als 100 µm zerkleinert.

6. Verfahren zur Herstellung einer schweißhemmenden Zubereitung feinteiliger adstringierender Aluminium-, Zirkonium- oder Zink-Salze, **dadurch gekennzeichnet, daß** man ein feinteiliges, adstringierendes Salz mit einer mittleren Teilchengröße von weniger als 100 µm mit einem flüssigen oder gelösten wasserlöslichen nichtionischen Tensid in einer Menge von 0,1 - 10 Gewichtsteilen des Tensids pro 100 Gewichtsteile des Aluminiumsalzes in der Wirbelschicht besprüht und gegebenenfalls das Lösungsmittel verdampft.

7. Verwendung der schweißhemmenden Zubereitungen gemäß Patentanspruch 1 - 4 als Komponente zur Herstellung von wasserfreien Antitranspirantien.

8. Antiperspirant in Form einer wasserfreien Zusammensetzung, **dadurch gekennzeichnet, daß** 0,1 - 40 Gew.-% einer schweißhemmenden Zubereitung gemäß einem der Ansprüche 1 - 4 darin dispergiert ist.

9. Antitranspirant gemäß Anspruch 8 in der Form eines Stiftes mit einem Gehalt von 10 - 60 Gew.-% einer unpolaren Trägerflüssigkeit und 5 - 30 Gew.-% einer verfestigenden Fettoder Wachskomponente, **dadurch gekennzeichnet, daß** 10 - 25 Gew.-% einer schweißhemmenden Zubereitung gemäß einem der Ansprüche 1 - 3 darin dispergiert sind.

## Claims

1. Perspiration-inhibiting preparations of fine-particle astringent aluminium, zirconium or zinc salts, **characterized in that** the particles have a mean particle size of less than 100 µm and are impregnated or coated with 0.1 to 10% by weight, based on the astringent aluminium, zirconium and zinc salts, of a water-soluble nonionic, zwitterionic or cationic surfactant.

2. Perspiration-inhibiting preparations as claimed in claim 1, **characterized in that** the aluminium hydroxychloride Al₂(OH)₅Cl · n H₂O, where n = 2 or 3, is present as the aluminium salt.

3. Perspiration-inhibiting preparations as claimed in claim 1, **characterized in that** the surfactant is an alkyl (oligo)glycoside with the formula R-O-(G)ₙ, in which R is an alkyl group containing 8 to 22 carbon atoms and (G)ₙ is the residue of a glucoside or oligoglucoside with an average degree of oligomerization n of 1 to 10.

4. Perspiration-inhibiting preparations as claimed in claim 1, **characterized in that** the surfactant is a dimethicone copolyol surfactant or a dimethicone polyglucoside surfactant which contains one or two polyoxyalkylene groups or glucoside units attached to a linear dimethyl polysiloxane chain.

5. A process for the production of a perspiration-inhibiting preparation of fine-particle astringent aluminium, zirconium or zinc salts, **characterized in that** the water present in an aqueous solution containing 100 parts by weight of the astringent salt and 0.1 to 10 parts by weight of a nonionic surfactant is removed and the residue is size-reduced to a mean particle size below 100 µm.

6. A process for the production of a perspiration-inhibiting preparation of fine-particle astringent aluminium, zirconium or zinc salts, **characterized in that** a fine-particle astringent salt with a mean particle size of less than 100 µm is sprayed with a liquid or dissolved, water-soluble nonionic surfactant in a quantity of 0.1 to 10 parts by weight of the surfactant to 100 parts by weight of the aluminium salt in a fluidized bed and the solvent is optionally evaporated.

7. The use of the perspiration-inhibiting preparations claimed in claims 1 to 4 as a component in the production of water-free antiperspirants.

8. An antiperspirant in the form of a water-free composition, **characterized in that** 0.1 to 40% by weight of the perspiration-inhibiting preparation claimed in any of claims 1 to 4 is dispersed therein.

9. An antiperspirant as claimed in claim 8 in the form of a stick containing 10 to 60% by weight of a nonpolar carrier liquid and 5 to 30% by weight of a solidifying fatty or wax component, **characterized in that** 10 to 25% by weight of the perspiration-inhibiting preparation claimed in any of claims 1 to 3 are dispersed therein.

## Revendications

1. Préparations inhibant la sudation, à base de sels d'aluminium, de zirconium ou de zinc finement divisés, astringents, **caractérisées en ce que** les particules présentent une taille moyenne inférieure à 100 µm et qu'elles sont imprégnées ou enrobées avec 0,1-10 % en poids, par rapport aux sels d'aluminium, de zirconium ou de zinc astringents, d'un tensioactif non ionique, zwitterionique ou cationique hydrosoluble.

2. Préparations inhibant la sudation selon la revendication 1, **caractérisées en ce que**, en tant que sel d'aluminium, elles contiennent de l'hydroxychlorure d'aluminium de formule Al₂(OH)₅Cl^{·}nH₂O, où n=2 ou 3.

3. Préparations inhibant la sudation selon la revendication 1, **caractérisées en ce que** le tensioactif est un alkyl-(oligo)-glycoside de formule R-O-(G)ₙ où R est un groupe alkyle comprenant 8 - 22 atomes de carbone et (G)ₙ est le reste d'un glucoside ou d'un oligo-glucoside ayant un degré moyen d'oligomérisation n de 1 - 10.

4. Préparations inhibant la sudation selon la revendication 1, **caractérisées en ce que** le tensioactif est un tensioactif diméthicone-copolyol ou un tensioactif diméthicone-polyglucoside qui contient un ou deux groupes polyoxyalkylène ou glucoside liés à une chaîne linéaire de diméthylpolysiloxane.

5. Procédé d'obtention d'une préparation inhibant la sudation à base de sels d'aluminium, de zirconium ou de zinc finement divisés, astringents, **caractérisé en ce que** l'on élimine l'eau d'une solution aqueuse ayant une teneur de 100 parties en poids d'un sel astringent et de 0,1 à 10 parties en poids d'un tensioactif non ionique et que l'on soumet le résidu à un broyage jusqu'à une taille moyenne des particules qui est inférieure à 100 µm.

6. Procédé d'obtention d'une préparation inhibant la sudation à base de sels d'aluminium, de zirconium ou de zinc finement divisés, astringents, **caractérisé en ce que** sur un sel astringent finement divisé, ayant une taille moyenne des particules inférieure à 100 µm, on pulvérise, dans la couche fluidisée, un tensioactif non ionique hydrosoluble en solution ou en liquide, à raison de 0,1 à 10 parties en poids de tensioactif pour 100 parties en poids de sel d'aluminium et éventuellement, on élimine le solvant par évaporation.

7. Utilisation des préparations inhibant la sudation selon les revendications 1-4, en tant que composant dans la préparation d'anti-transpirants anhydres.

8. Anti-transpirant sous la forme d'une composition anhydre, **caractérisé en ce qu'**il contient, sous forme de dispersion, 0,1 - 40 % en poids d'une préparation inhibant la sudation selon l'une quelconque des revendications 1 à 4.

9. Anti-transpirant selon la revendication 8 sous la forme d'un stick, ayant une teneur de 10 - 60 % en poids d'un véhicule liquide non polaire et de 5 - 30 % en poids d'un composant solidifiant de type graisse ou cire, **caractérisé en ce qu'**il contient, sous forme de dispersion, 10-25% en poids d'une préparation inhibant la sudation selon l'une quelconque des revendications 1 à 3.
